# EUROPEAN PATENT APPLICATION

(11) **EP 4 678 160 A1**
(43) Date of publication of application: **14.01.2026**
(21) Application number: 24187903.0
(22) Date of filing: 11.07.2024
(51) Int. Cl.: A61K 8/66, A61Q 5/00, A61Q 17/00

(54) **A HAIR AND SCALP MICROBIOME-FRIENDLY COMBINATION OF ENZYMES**

(71) Applicant: SkyLab AG, 1066 Epalinges (CH)
(72) Inventor: Belous, Elena Yur'evna, 121309 MOSCOW (RU); Ivanova, Angelina Dmitrievna, London, NW61NE (GB)
(74) Representative: Glawe, Delfs, Moll

(57) **Abstract**

The invention is related to specific enzyme combinations for use in the field of the hair and scalp care products.

## Description

### FIELD OF INVENTION

The present invention is related to the novel uses of chitinase with chitosanase and can be useful in hair care products.

### BACKGROUND OF INVENTION

The human skin is inhabited by thousands of microorganisms, which form so-called "skin flora". Microbiome means the genome of all microorganisms, both symbiotic and pathogenic, which interact with all types of cells in the organism [1]. Metabolic exchanges between the scalp surface and microbiome generally support symbiotic, commensal or pathogenic growth of microbial biofilms [2,3]. In the recent years a lot of scientific reports on the studies of microbial population were published, in particularly, about scalp inhabiting bacteria and fungi [4-9]. Of note, *Malassezia, Staphylococcus, Aspergillus, Pseudomonas* spp., etc., are the most important scalp pathogens [15]. The commensal can turn to causative pathogens, so none of the microorganisms can be considered as definitely beneficial. *S*. *epidermidis* and *C*. *albicans* are among the most thoroughly studied and well known examples [16].

As reported in the studies [10-13], *P. acnes* and *S. epidermidis* were found in the main bacterial communities. In particular, in the scalp with dandruff *P. acnes*/*S. epidermidis* ratio was found to be lower than in healthy scalp. *Malassezia restricta* and *Malassezia globosa* appeared to be the main fungal scalp colonizers, and lower *M. restricta*/*M. globosa* ratio was associated with a healthy scalp. Therefore, the dandruff scalp microbiome is characterized by higher population of *non-cultured Malassezia sp.* compared to the healthy scalp [14].

Numerous antifungal systems are used for the treatment of fungal infections. For example, climbazole is one of the novel antifungal agents usually used in combination or as monotherapy. Climbazole affects normal functioning of the fungal cell wall leading to anti-fungal activity, however, this agent can cause local skin irritation manifested by the symptoms including redness, rash, and pruritus, and also allergic reactions [17].

Selenium sulfide eliminates excessive hair loss and irritation caused by the fungal scalp infection and reduces the cell adhesion in corneal layer. This agent possesses also local irritative, antibacterial, and light anti-fungal activities, which can contribute to its efficacy. Potential side effects include incidental greasiness or dryness of hair or scalp, greater hair loss, and scalp discoloration [18].

Piroctone olamine is used in shampoos due to its antifungal activity. This agent inhibits the degradation of sebum triglycerides to oleic and arachidonic acids. Side effects include irritation, edema, numbness, skin hemorrhage and shrinkage [19].

Zinc pyrithione is the most popular antifungal agent (active against the most common scalp-inhabiting *Malassezia* species *(Malassezia globosa* 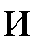 *Malassezia restrica*), which is used as a component of various formulations for dandruff treatment. Despite many other systems zinc pyrithione is free of general side effects (though can cause harm upon ingestion) [17]. The efficacy depends on the targeted delivery of zinc pyrithione to the skin areas inhabited by these yeast species, including the scalp surface and the stalk of hair follicle [20]. Zinc pyrithione inhibits the membrane transport processes and cell division in the fungal cell wall [21]. Since March 1, 2022, the use of zinc pyrithione in the beauty products available in EU markets has been prohibited. The European Union decided to add this ingredient into the prohibited agent list because of its DNA-damaging effect.

As is known the fungi of Malassezia spp. are the main causative agents of dandruff of *Malassezia* genus.

To date seven Malassezia species have been found and identified: *M. furfur, M. sympodialis, M. obtusa, M. globosa, M. restricta, M. slooffiae* and *M. pachydermatis [28].*

The fungal cell wall is composed of mannoproteins, chitin, and β-glucancs.

Thomas Stalhberger *et al.* [36] reported that *Malassezia* species are ubiquitous residents of human skin and are associated with several diseases such as seborrheic dermatitis, tinea versicolor, folliculitis, atopic dermatitis, and scalp conditions such as dandruff. Host-*Malassezia* interactions and mechanisms to evade local immune responses remain largely unknown. *Malassezia restricta* is one of the most predominant yeasts of the healthy human skin, Polysaccharides in the *M*. *restricta* cell wall are almost exclusively alkali-insoluble, showing that they play an essential role in the organization and rigidity of the *M. restricta* cell wall. Fractionation of cell wall polymers and carbohydrate analyses showed that the polysaccharide core of the cell wall of *M. restricta* contained an average of 5% chitin, 20% chitosan, 5% β-(1.3)-glucan, and 70% β-(1.6)-glucan. In contrast to other yeasts, chitin and chitosan are relatively abundant, and β-(1.3)-glucans constitute a minor cell wall component. The most abundant polymer is β-(1.6)-glucans, which are large molecules composed of a linear β-(1.6)-glucan chains with β-(1.3)-glucosyl side chain with an average of 1 branch point every 3.8 glucose unit. Both β-glucans are cross-linked, forming a huge alkali-insoluble complex with chitin and chitosan polymers. *M. restricta* has a polysaccharide organization very different of all fungal species analyzed to date. The average molecular mass of β-(1.6)-glucan chain about 80 kDa (around 500 glucose units/chain). In contrast to most fungi analyzed, the polysaccharide core of the *M. restricta* cell wall was extremely alkali-insoluble. HPLC and GLC analysis of sugar composition showed that both AS and AI fractions were composed of glucose and glucosamine residues only, at 94/6 and 75/25 ratios, respectively, suggesting the presence of glucans and chitin/chitosan in the cell wall. To identify cell wall polysaccharides, specific enzymatic digestions were performed on the AI fraction. Recombinant endo-β-(1.3)-glucanase, endo-β-(1.6)-glucanase, chitosanase, and chitinase partially degraded the AI fraction, indicating the presence of β-(1.3)-glucan, β-(1.6)-glucan, chitin, and chitosan. *M. restricta M. restricta* is a commensal yeast of the skin microbiota. Under steady state conditions, *Malassezia* yeasts manage to evade local immune responses and remain in balance with the human skin. Because fungal cell wall carbohydrates are widely conserved across the fungal kingdom, but absent in humans, these structural components of the cell wall are convenient targets for immune recognition and critical for host inflammation responses. β-(1.3)-Glucan recognition has been extensively studied as receptors have been identified in a number of human cells including keratinocytes (*e*.*g*. langerrin, dectin-1. CR3). In contrast, the effect of β-(1.6)-glucans on the immune system has been poorly studied. Chitin is recognized by the immune system and particularly the keratinocytes. Chitin and chitosan were also activators of the human immune system, but their recognition and signaling pathways are totally independent. In addition, it has also been observed that large chitin fragments or the conversion of chitin to chitosan favors the escape from immune recognition. The characterization of the cell wall polysaccharides paves the way for a thorough understanding of the role of *M. restricta* polysaccharides (specially β-(1.6)-glucan or chitosan) in local immune response or in masking minor polysaccharides (*e.g.* β-(1.3)-glucan and chitin) from host recognition.

Another species of *Malassezia genus* - *M. furfur* is a polymorphic lipophilic microorganism characterized by a thick, multilayer cell wall composed at an average of 5% of chitin, 20% of chitosan, 5% β-(1,3)-glucan and 70% of β-(1,6)-glucan [22]. It was shown that BuGH30 (glycosyl hydrolase degrades predominantly long-chain β-1-6 glucans with gentiobiose being a final product [23]. In addition, several enzymes related to chitin were found, namely, putative chitinases falling into GH 18 family, exochitinase in GH20 family and putative chitosanase in GH 75 family[24]. Chitosan can be enzymatically degraded by chitinases and chitosanases [25].

Chitinases are the enzymes degrading chitin. Chitinases are widely used, for instance, to produce pharmaceutically important chito-oligosaccharides and N-acetyl-D-glycosamine, a single-cell protein, to isolate the protoplasts from fungi and yeast, and to control the fungal pathogens [26]. Chitinase is the most active at temperature 60°C amd pH=5,5 [DOI:10.21161/mjm.44612]. Chitosanases are glycosyl hydrolases catalyzing the endohydrolysis of β-1,4-glycoside bonds in partially acetylated chitosan leading to release of the chitosan oligosaccharides. Chitosanases from various sources, mainly bacterial and fungal chitosanases, have been isolated, purified and characterized. Chitosanases became very attractive because of the broad range of potential uses as an advantageous agent for biocontrol of pathogenic fungi and insects, bioconversion of chitin biowastes resulted from the sea foods processing, etc. [27]. The optimal environmental conditions favoring the enzyme activity are 55°C and pH 5 [Zhang, Q., Cao, H. Expression of chitosanase from Aspergillus fumigatus chitosanase in Saccharomyces cerevisiae by CRISPR-Cas9 tools. Bioresour. Bioprocess. 11. 20 (2024). DOI: 10.1186/s40643-023-00718-4].

Chitosanase and chitinase partially degraded the cell wall of *M. furfur.* Enzymatic degradation by chitosanase resulted in solution of 75% of glucosamine and conversion of alkali-insoluble glucan fraction to water-soluble glucans [22].

Cellulase is an hydrolase catalyzing the cleavage of β(1,4)-glycosidic bonds in cellulose giving rise to glucose or cellobiose disaccharide. Cellulase degrades the cell wall component (cellulose) leading to release of biologically active compounds [29]. The highest efficacy is observed at 50-55°C and pH 5.5 [DOI:10.2478/mjhr-2019-0009]. Cellulases are exist as a multicomponent enzymatic system generally comprising three enzymes, which synergistically degrade the cellulose: endoglucanase, cellobiohydrolase and cellobiase (β-glucosidase). The first two enzymes work directly upon cellulose giving rise predominantly cellobiose and glucose as the reaction products, followed by glucose degradation by cellobiase [30].

β-D-mannanases are endohydrolases, which randomly cleave a backbone of 1,4-β-D-mannane galactomannane, glycomannane, galactoglucomannane, and mannane [31], with the highest activity achieved at pH 6.0 and 65°C [DOI:10.1155/2016/6380147]. β-mannanase has a strong potential to be used in many industrial fields including feed, food, pharmaceutical and beauty industries [32].

Glucanases are the enzymes degrading high molecular weight polysaccharides through hydrolysis. The hydrolysis product is called glucan, which is a linear polysaccharide comprising up to 1200 monomeric glucose residues linked by glycosidic bonds. Catalysis of glycosidic bonds in polysaccharides is main function of glucanase. This function is no highly specific, and substrate specificity of the enzymes is based mainly on the bond types and α- or β-configuration [33]. β-1,3-Glucanases are able to destroy the cell wall of some fungi and to cleave the fragments exhibiting immunoregulatory activities (elicitors and suppressors) [34], with the highest activity achieved at pH 4.0 and 80 °C [DOI: 10.1111/j. 1750-3841.2007.00549.x],

Beta-glucosidase, a group of glycosyl hydrolases commonly found in fungi, plants and bacteria, have identical structures and sequences. β-glucosidases producing monosaccharides from cellulose-based oligosaccharides play a key role in the biomass hydrolysis. The highest activity is achieved at 85 °C, pH 4.5-7.0 [Singhania RR, Patel AK, Sukumaran RK, Larroche C, Pandey A. Role and significance of beta-glucosidases in the hydrolysis of cellulose for bioethanol production. Bioresour Technol. 2013 Jan;127:500-7. DOI:10.1016/j.biortech.2012.09.012]. According to classification based on substrate specificity β-glucosidases fall into three groups: aryl-β-glucosidase cleaving predominantly aryl glucosides; cellobiases involved in disaccharide cellobiose transformation to glucose; and glucosidases, which are most abundant and demonstrate extensive substrate-specific activities toward a broad range of substrates [35].

Different environmental conditions required for optimal enzyme activity could be the main factor limiting the early development of such enzymatic system.

Some formulations of anti-dandruff shampoos are known from [37]. The authors reported that *Malassezia* species are common, clinically relevant, and lipid-dependent yeasts of humans. They are also the leading causes of the dandruff problem of humans, and the azoles are used primarily in their topical and systemic treatment. Resistance to azoles is an emerging problem among *Malassezia* spp., which limits the use of azoles and other agents in treatment. The efficacy of various combinations of piroctone olamine and climbazole is highly important. The disclosed study included nine formulations, where each formulation was prepared from different concentrations of piroctone olamine and climbazole and both. All formulations contained the same ingredients: water, surfactants, hair conditioning agents, and preservatives. *Malassezia furfur* CBS1878, *Malassezia globosa* CBS7874, and *Malassezia sympodialis* CBS9570 were tested for antifungal susceptibility of each formulation by agar diffusion method. Sizes of the inhibition zones were compared with standard medical shampoo containing 2% ketoconazole, and the data were analyzed by Dunnett's multiple-comparison test. For all *Malassezia* sp. strains, climbazole 0.5% and piroctone olamine/climbazole (0.1%/0.1% and 0.1%/0.5%) combinations were found to have the same effect as the medical shampoo containing 2% ketoconazole. Piroctone olamine/climbazole 1.0%/0.1% formulation showed the same efficacy as 2% ketoconazole on *M. furfur* and *M*. *sympodialis,* while 0.1%/0.5% formulation was effective only against *M*. *furfur. For M. globosa,* none of the formulations tested was as effective as ketoconazole.

The use of chitosanase in preparation of formulations intended for treatment or prevention of skin diseases caused by *Malassezia* spp. is disclosed in Chinese patent application [38]. The authors provided the method of preparation of chitosanase composite gel, comprising the following steps: 1) dissolution of chitosanase in acetic acid-sodium acetate buffer; 2) dissolution of hyaluronic acid in acetic acid-sodium acetate buffer; 3) slow addition of chitosanase solution to hyaluronic acid solution with stirring to obtain the resultant product.

However, there remains a need in highly effective products, which will be able to destroy the fungi of *Malassezia* spp. causing dandruff.

### SUBSTANCE OF THE INVENTION

The invention is set out in the appended claims. The following embodiments show the substance of the invention. It will be understood by those skilled in the art that possible small variations of the listed embodiments are nevertheless also within the scope of the invention.
1. The use of combination of two enzymes selected from chitinase and chitosanase to prepare a therapeutic anti-dandruff head-washing shampoo.
2. The use of combination of two enzymes selected from chitinase and β-glucosidase to prepare a therapeutic anti-dandruff head-washing shampoo.
3. The use of combination of two enzymes selected from chitinase and β-glucanase to prepare a therapeutic anti-dandruff head-washing shampoo.
4. The use of combination of two enzymes selected from chitinase and β-mannanase to prepare a therapeutic anti-dandruff head-washing shampoo.
5. The use of combination of two enzymes selected from chitinase and cellulase to prepare a therapeutic anti-dandruff head-washing shampoo.
6. The use according to any one of embodiments 1 to 5, wherein chitinase and the second enzyme of said combination are taken at a mass ratio 1:(0.5-1.5), preferably 1:(0.7-1.3), more preferably 1:(0.9-1.1), even more preferably 1:1.
7. The use of chitinase in combination with two or more enzymes selected from the group comprising chitosanase, β-glucosidase, β-glucanase, β-mannanase and cellulase, to prepare a therapeutic anti-dandruff head-washing shampoo.
8. The use according to embodiment 7, wherein chitinase and the second enzyme of said combination are taken at a mass ratio 1:(0.5-1.5), preferably 1:(0.7-1.3), more preferably 1:(0.9-1.1), even more preferably 1:1.
9. The use according to embodiment 7, wherein chitinase, the second enzyme and the third enzyme of said combination are taken at a mass ratio 1:(0.5-1.5):(0.5-1.5), preferably 1:(0.7-1.3):(0.7-1.3), more preferably 1:(0.9-1.1):(0.9-1.1), even more preferably 1:1:1.
10. The use according to embodiment 7, wherein chitinase, the second enzyme, the third enzyme, and the fourth enzyme of said combination are taken at a mass ratio 1:(0.5-1.5):(0.5-1.5):(0.5-1.5), preferably 1:(0.7-1.3):(0.7-1.3):(0.7-1.3), more preferably 1:(0.9-1.1):(0.9-1.1):(0.9-1.1), even more preferably 1:1:1:1.
11. The use according to embodiment 7, wherein chitinase, the second enzyme, the third enzyme, the fourth enzyme, and the fifth enzyme of said combination are taken at a mass ratio 1:(0.5-1.5):(0.5-1.5):(0.5-1.5):(0.5-1.5), preferably 1:(0.7-1.3):(0.7-1.3):(0.7-1.3):(0.7-1.3), more preferably 1:(0.9-1.1):(0.9-1.1):(0.9-1.1):(0.9-1.1), even more preferably 1:1:1:1:1.
12. The use according to embodiment 7, wherein chitinase, the second enzyme, the third enzyme, the fourth enzyme, the fifth enzyme, and the sixth enzyme of said combination are taken at a mass ratio 1:(0.5-1.5):(0.5-1.5):(0.5-1.5):(0.5-1.5):(0.5-1.5), preferably 1:(0.7-1.3):(0.7-1.3):(0.7-1.3):(0.7-1.3):(0.7-1.3), more preferably 1:(0.9-1.1):(0.9-1.1):(0.9-1.1):(0.9-1.1):(0.9-1.1), even more preferably 1:1:1:1:1:1.
13. The use of chitosanase in combination with two or more enzymes selected from the group comprising chitinase, β-glucosidase, β-glucanase, β-mannanase, and cellulase to prepare a therapeutic anti-dandruff head-washing shampoo.
14. The use according to embodiment 13, wherein chitosanase and the second enzyme of said combination are taken at a mass ratio 1:(0.5-1.5), preferably 1:(0.7-1.3), more preferably 1:(0.9-1.1), even more preferably 1:1.
15. The use according to embodiment 13, wherein chitosanase, the second enzyme and the third enzyme of said combination are taken at a mass ratio 1:(0.5-1.5):(0.5-1.5), preferably 1:(0.7-1.3):(0.7-1.3), more preferably 1:(0.9-1.1):(0.9-1.1), even more preferably 1:1:1.
16. The use according to embodiment 13, wherein chitosanase, the second enzyme, the third enzyme and the fourth enzyme of said combination are taken at a mass ratio 1:(0.5-1.5):(0.5-1.5):(0.5-1.5), preferably 1:(0.7-1.3):(0.7-1.3):(0.7-1.3), more preferably 1:(0.9-1.1):(0.9-1.1):(0.9-1.1), even more preferably 1:1:1:1.
17. The use according to embodiment 13, wherein chitosanase, the second enzyme, the third enzyme, the fourth enzyme 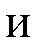 the fifth enzyme of said combination are taken at a mass ratio 1:(0.5-1.5):(0.5-1.5):(0.5-1.5):(0.5-1.5), preferably 1:(0.7-1.3):(0.7-1.3):(0.7-1.3):(0.7-1.3), more preferably 1:(0.9-1.1):(0.9-1.1):(0.9-1.1):(0.9-1.1), even more preferably 1:1:1:1:1.
18. The use according to embodiment 13, wherein chitosanase, the second enzyme, the third enzyme, the fourth enzyme, the fifth enzyme 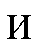 the sixth enzyme of said combination are taken at a mass ratio 1:(0.5-1.5):(0.5-1.5):(0.5-1.5):(0.5-1.5):(0.5-1.5), preferably 1:(0.7-1.3):(0.7-1.3):(0.7-1.3):(0.7-1.3):(0.7-1.3), more preferably 1:(0.9-1.1):(0.9-1.1):(0.9-1.1):(0.9-1.1):(0.9-1.1), even more preferably 1:1:1:1:1:1.
19. The use of cellulase in combination with two or more enzymes selected from the group comprising chitosanase, chitinase, β-glucosidase, β-glucanase, and β-mannanase, to prepare a therapeutic anti-dandruff head-washing shampoo.
20. The use according to embodiment 19, wherein cellulase and the second enzyme of said combination are taken at a mass ratio 1:(0.5-1.5), preferably 1:(0.7-1.3), more preferably 1:(0.9-1.1), even more preferably 1:1.
21. The use according to embodiment 19, wherein cellulase, the second enzyme and the third enzyme of said combination are taken at a mass ratio 1:(0.5-1.5):(0.5-1.5), preferably 1:(0.7-1.3):(0.7-1.3), more preferably 1:(0.9-1.1):(0.9-1.1), even more preferably 1:1:1.
22. The use according to embodiment 19, wherein cellulase, the second enzyme, the third enzyme and the fourth enzyme of said combination are taken at a mass ratio 1:(0.5-1.5):(0.5-1.5):(0.5-1.5), preferably 1:(0.7-1.3):(0.7-1.3):(0.7-1.3), more preferably 1:(0.9-1.1):(0.9-1.1):(0.9-1.1), even more preferably 1:1:1:1.
23. The use according to embodiment 19, wherein cellulase, the second enzyme, the third enzyme, the fourth enzyme 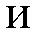 the fifth enzyme of said combination are taken at a mass ratio 1:(0.5-1.5):(0.5-1.5):(0.5-1.5):(0.5-1.5), preferably 1:(0.7-1.3):(0.7-1.3):(0.7-1.3):(0.7-1.3), more preferably 1:(0.9-1.1):(0.9-1.1):(0.9-1.1):(0.9-1.1), even more preferably 1:1:1:1:1.
24. The use according to embodiment 19, wherein cellulase, the second enzyme, the third enzyme, the fourth enzyme, the fifth enzyme 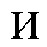 the sixth enzyme of said combination are taken at a mass ratio 1:(0.5-1.5):(0.5-1.5):(0.5-1.5):(0.5-1.5):(0.5-1.5), preferably 1:(0.7-1.3):(0.7-1.3):(0.7-1.3):(0.7-1.3):(0.7-1.3), more preferably 1:(0.9-1.1):(0.9-1.1):(0.9-1.1):(0.9-1.1):(0.9-1.1), even more preferably 1:1:1:1:1:1.
25. The use according to any one of embodiments 1 to 24, wherein the dandruff is caused by the presence of at least one of *M. furfur, M. sympodialis, M. obtusa, M. globosa, M. restricta, M. slooffiae* and/or *M. pachydermatis* on scalp.
26. The use according to embodiment 25, wherein the dandruff is caused by the presence of *M. furfur* on scalp.
27. The use according to embodiment 25, wherein the dandruff is caused by the presence of *M. sympodialis* on scalp.
28. The use according to embodiment 25, wherein the dandruff is caused by the presence of *M. obtuse* on scalp.
29. The use according to embodiment 25, wherein the dandruff is caused by the presence of *M. globose* on scalp.
30. The use according to embodiment 25, wherein the dandruff is caused by the presence of *M. restricta* on scalp.
31. The use according to embodiment 25, wherein the dandruff is caused by the presence of *M. slooffiae* on scalp.
32. The use according to embodiment 25, wherein the dandruff is caused by the presence of *M. pachydermatis* on scalp.
33. The use according to any one of the embodiments 1 to 24, wherein the dandruff is caused by the presence of any two of *M. furfur, M. sympodialis, M. obtusa, M. globosa, M. restricta, M. slooffiae* and/or *M. pachydermatis* on scalp.
34. The use according to any one of the embodiments 1 to 24, wherein the dandruff is caused by the presence of any three of *M. furfur, M. sympodialis, M. obtusa, M. globosa, M. restricta, M. slooffiae* and/or *M. pachydermatis* on scalp.
35. The use according to any one of the embodiments 1 to 24, wherein the dandruff is caused by the presence of any four of *M. furfur, M. sympodialis, M. obtusa, M. globosa, M. restricta, M. slooffiae* and/or *M. pachydermatis* on scalp.
36. The use according to any one of the embodiments 1 to 24, wherein the dandruff is caused by the presence of any five of *M. furfur, M. sympodialis, M. obtusa, M. globosa, M. restricta, M. slooffiae* and/or *M. pachydermatis* on scalp.
37. The use according to any one of the embodiments 1 to 24, wherein the dandruff is caused by the presence of any six of *M. furfur, M. sympodialis, M. obtusa, M. globosa, M. restricta, M. slooffiae* and/or *M. pachydermatis* on scalp.
38. The use according to any one of the embodiments 1 to 24, wherein the dandruff is caused by the presence of *M. furfur, M. sympodialis, M. obtusa, M. globosa, M. restricta, M. slooffiae* and *M. pachydermatis* on scalp.

### DETAILED DESCRIPTION OF THE INVENTION

The present invention is related to the use of combination of two enzymes selected from chitinase and chitosanase to prepare a therapeutic anti-dandruff head-washing shampoo.

The present invention is also related to the use of combination of two enzymes selected from chitinase and β-glucosidase to prepare a therapeutic anti-dandruff head-washing shampoo.

The present invention is also related to the use of combination of two enzymes selected from chitinase and β-glucanase to prepare a therapeutic anti-dandruff head-washing shampoo.

The present invention is also related to the use of combination of two enzymes selected from chitinase and β-mannanase to prepare a therapeutic anti-dandruff head-washing shampoo.

The present invention is also related to the use of combination of two enzymes selected from chitinase and cellulase to prepare a therapeutic anti-dandruff head-washing shampoo.

The present invention is also related to the use of chitinase in combination with two or more enzymes selected from the group comprising chitosanase, β-glucosidase, β-glucanase, β-mannanase, and cellulase to prepare a therapeutic anti-dandruff head-washing shampoo.

The present invention is also related to the use of chitosanase in combination with two or more enzymes selected from the group comprising chitinase, β-glucosidase, β-glucanase, β-mannanase, and cellulase to prepare a therapeutic anti-dandruff head-washing shampoo.

The present invention is also related to the use of cellulase in combination with two or more enzymes selected from the group comprising chitosanase, chitinase, β-glucosidase, β-glucanase, and β-mannanase to prepare a therapeutic anti-dandruff head-washing shampoo.

The dandruff can be caused by the presence on scalp of at least any one of *M. furfur, M. sympodialis, M. obtusa, M. globosa, M. restricta, M. slooffiae* and/or *M. pachydermatis.*

More particularly, the dandruff can be caused by the presence of *M. furfur* on scalp. More particularly, the dandruff can be caused by the presence of *M. sympodialis* on scalp. More particularly, the dandruff can be caused by the presence of *M. obtuse* on scalp. More particularly, the dandruff can be caused by the presence of *M. globose* on scalp. More particularly, the dandruff can be caused by the presence of *M. restricta* on scalp. More particularly, the dandruff can be caused by the presence of *M. slooffiae* on scalp. More particularly, the dandruff can be caused by the presence of *M. pachydermatis* on scalp.

More particularly, the dandruff can be caused by the presence of any two of *M. furfur, M. sympodialis, M. obtusa, M. globosa, M. restricta, M. slooffiae* and/or *M. pachydermatis* on scalp.

More particularly, the dandruff can be caused by the presence of any three of *M. furfur, M. sympodialis, M. obtusa, M. globosa, M. restricta, M. slooffiae* and/or *M. pachydermatis* on scalp.

More particularly, the dandruff can be caused by the presence of any four of *M. furfur, M. sympodialis, M. obtusa, M. globosa, M. restricta, M. slooffiae* and/or *M. pachydermatis* on scalp.

More particularly, the dandruff can be caused by the presence of any five of *M. furfur, M. sympodialis, M. obtusa, M. globosa, M. restricta, M. slooffiae* and/or *M. pachydermatis.*

More particularly, the dandruff can be caused by the presence of any six of *M. furfur, M. sympodialis, M. obtusa, M. globosa, M. restricta, M. slooffiae* and/or *M. pachydermatis.*

More particularly, the dandruff can be caused by the presence of *M. furfur, M. sympodialis, M. obtusa, M. globosa, M. restricta, M. slooffiae* 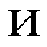 *M. pachydermatis* on scalp.

In accordance with invention, chitinase, or chitosanase, or cellulase, and the second enzyme, and/or the third enzyme, and/or the fourth enzyme, and/or the fifth enzyme and/or the sixth enzyme (if present in the embodiment of combination) can be taken at a mass ratio 1 :(0.5-1.5):(0.5-1.5):(0.5-1.5):(0.5-1.5):(0.5-1.5), preferably 1:(0.7-1.3):(0.7-1.3):(0.7-1.3):(0.7-1.3):(0.7-1.3), more preferably 1:(0.9-1.1):(0.9-1.1):(0.9-1.1):(0.9-1.1):(0.9-1.1), even more preferably 1:1:1:1:1:1 (depending on the number of taken enzymes).

In accordance with invention, the enzyme concentration can be in the range from 0.05 to 5 % by mass including 0.05, 0.1. 0.125, 0.2, 0.25, 0.3, 0.4, 0.5, 0.6, 0.7, 0.8, 0.9, 1.0. 1.2, 1.4, 1.6, 1.8, 2,0. 2.2, 2.4, 2.6, 2.8, 3.0. 3.2, 3.4, 3.6, 3.8, 4.0. 4.2, 4.4, 4.6, 4.8, 5.0, and in any range between the listed values.

### EXPERIMENT

Commercially available products containing climbazole, piroctone olamine, selenium sulfide, zinc pyrithione and the enzymes are listed in Tables 1 and 2.

**Table 1. Commercially available products**

| No. | Ingredient | CAS Number | Origin | Supplier |
|---|---|---|---|---|
| 1 | Climbazole Crinipan^{®} AD | 38083-17-9 | Synthetic | Symrise, Germany |
| 2 | Piroctone olamine Cosroma^{®} OCT | 68890-66-4 | Synthetic | COSROMA, China |
| 3 | Selenium sulfide Cosroma^{®} SS20 | 7488-56-4 | Synthetic | COSROMA, China |
| 4 | Zinc pyrithione | 13463-41-7 | Synthetic | Salicylates and Chemicals, India |

**Table 2. Investigated enzymes for innovative system**

| No. | Enzyme | CAS Number | Class | Appearance | Composition | Supplier |
|---|---|---|---|---|---|---|
| 1 | Chitinase | 9001-06-3 | 3.2.1 Glycosidases | Brownish yellow powder | 30% Enzyme 70% Magnesium sulfate | Shaanxi Pioneer Biotech Co.,Ltd China |
| 2 | Chitosanase | 51570-20-8 | 3.2.1 Glycosidases | Brownish yellow powder | 30% Enzyme 70% Magnesium sulfate | |
| 3 | β-glucanase | 62213-14-3 | 3.2.1 Glycosidases | Light yellow powder | 8% Enzyme 92% Magnesium sulfate | SunSon Industry Group Co., Ltd., China |
| 4 | β-glucosidase | 9001-22-3 | 3.2.1 Glycosidases | Brown liquid | 10% Enzyme 90% Water | |
| 5 | Cellulase | 9012-54-8 | 3.2.1 Glycosidases | Brown liquid | 8% Enzyme 92% Water | |
| 6 | β-mannanase | 37288-54-3 | 3.2.1 Glycosidases | Brown liquid | 8% Enzyme 92% Water | Wu Xi Green Year Union Works Co., Ltd, China |

### Test Microorganisms and Growth Conditions

The fungal strains: *M. furfur CBS 1878, M. restricta CBS 7877, M. globosa CBS 7966* were purchased from Westerdijk institute, Utrecht, The Netherlands. The strains were cultured in the The Sabouraud dextrose agar (SDA) agar and then incubated in the CHROMagar Malassezia^{®} with Tween-40 and Glycerol for visible growth at 37 °C for 72 h. The inoculum was diluted and cells were counted in a Neubauer chamber to reach desirable inoculum concentrations 1.0 × 106 CFU/mL

All microbiology work was done inside a microbiology safety cabinet (Faster, Italy).

### Antifungal Screening by Determining the Log10CFU Reduction

The Log10CFU reduction in fungi in the presence of antifungal substances was used to evaluate the inhibitory effect of the tested substance on a population of *M. furfur, M. globosa, M. restricta* by exposure to the substance through quantitate evaluation of viable total colony counts. The final concentrations of substances were 5.00, 3.00, 2.00, 1.00, 0.50, 0.25, 0.125 and 0.05 weight % prepared in water with 0.3% Polysorbate 80. Antifungal drugs such as climbazole, piroctone olamine, zinc pyrithione and selenium sulfide, recommended for the treatment of dandruff, were used as controls. Then, 1 mL of microorganisms at a 1.0 × 106 CFU/mL concentration was cultured with 9 mL of each sample concentration. The experimental procedure consisted of the substance incubation with a known suspension of *M*. *furfur, M. restricta and M. globosa* at 37 °C for 1 hour as the average time for a possible effect of ingrediets for the treatment of dandruff. The incubation was performed on substrate culture media (Mueller Hinton broth). After the incubation period, the viable fungi population was monitored by counting the viable cells on an agar plate (CHROMagar Malassezia). The suspension of the microorganism inoculum without substance was used as a negative control. The experiments were carried out in triplicate. The result was expressed in a decimal logarithm of the reduction in colony forming units per mL (log10CFU/mL) (Table 3) and then converted to anti-fungal efficacy indicated in percentage of inhibition (Table 4). Inhibitory activity of the enzymes against Malassezia spp. was confirmed by stability results at temperatures from 4 to 40 °C. The pH of the medium was typical of hair products and was between 4 and 8.

**Table 3. Results of determining the Log10CFU Reduction**

| *No.* | *Strains (M. furfur CBS 1878, M. restricta CBS 7877, M. globosa CBS 7966 1.0* × *106 CFU*/*mL)* | *Log10CFU, Mean* ± *SD ^{a}* | | | | | | | |
|---|---|---|---|---|---|---|---|---|---|
| | | Negative control | 0.05% | 0.125% | 0.25% | 0.5% | 1.00% | 3.00% | 5.00% |
| *1* | Climbazole | | 0.61 | 0.77 | 0.8 | 0.89 | 0.93 | 1.14 | >5.85 |
| *2* | Piroctone olamine | | 1.19 | 1.63 | >5.85 | >5.85 | >5.85 | >5.85 | >5.85 |
| *3* | Selenium sulfide | | 1.41 | 1.83 | >5.85 | >5.85 | >5.85 | >5.85 | >5.85 |
| *4* | Zinc pyrithione | | 0.87 | 1.36 | >5.85 | >5.85 | >5.85 | >5.85 | >5.85 |
| *5* | Chitinase | | 0.04 | 0.08 | 0.12 | 0.19 | 0.25 | 0.42 | 0.44 |
| *6* | Chitosanase | | 0.01 | 0.02 | 0.13 | 0.14 | 0.27 | 0.43 | 0.49 |
| *7* | Chitinase + Chitosanase (ration 1:1) | | 0.77 | 1.04 | 1.79 | >5.85 | >5.85 | >5.85 | >5.85 |
| *8* | β-glucanase | | 0.01 | 0.02 | 0.05 | 0.06 | 0.10 | 0.16 | 0.32 |
| *9* | β-glucosidase | | 0.05 | 0.09 | 0.17 | 0.21 | 0.25 | 0.39 | 0.56 |
| *10* | β-mannanase | | 0.005 | 0.012 | 0.015 | 0.016 | 0.019 | 0.03 | 0.04 |
| *11* | Cellulase | | 0.06 | 0.07 | 0.12 | 0.13 | 0.19 | 0.22 | 0.32 |
| *12* | Chitinase + β-glucanase (ratio 1:1) | | 0.51 | 0.61 | 0.75 | 0.84 | 1.03 | >5.85 | >5.85 |
| *13* | β-mannanase + β-glucosidase + chitinase (ratio 1:1:1) | | 1.35 | 1.71 | >5.85 | >5.85 | >5.85 | >5.85 | >5.85 |
| *14* | Chitosanase + β-glucosidase + chitinase (ratio 1:1:1) | | 2.35 | 2.52 | >5.85 | >5.85 | >5.85 | >5.85 | >5.85 |
| *15* | Chitinase + Cellulase (ratio 1:1) | | 0.49 | 0.53 | 0.86 | 0.89 | 1.26 | 1.52 | >5.85 |
| *16* | Chitinase + β-glucosidase (ratio 1:1) | | 0.76 | 0.82 | 0.91 | 0.99 | 1.10 | 1.20 | >5.85 |
| *17* | Chitinase + β-mannanase (ratio 1:1) | | 0.62 | 0.69 | 0.70 | 0.71 | 0.76 | 0.77 | 0.81 |
| *18* | Chitosanase + β-glucosidase (ratio 1:1) | | 0.68 | 0.85 | 0.91 | 1.19 | 2.03 | 3.69 | >5.85 |
| *19* | Chitosanase + β-glucanase (ratio 1:1) | | 0.32 | 0.43 | 0.53 | 0.62 | 0.75 | 0.85 | 0.99 |

**Table 4. Percent of Malassezia spp. inhibition**

| **Concentration** | **0.05%** | **0.125%** | **0.25%** | **0.5%** | **1.00%** | **3.00%** | **5.00%** |
|---|---|---|---|---|---|---|---|
| | % of Malassezia spp. inhibition | | | | | | |
| Climbazole | 75.45 | 83.02 | 84.43 | 87.26 | 88.21 | 92.65 | 100.00 |
| Piroctone olamine | 93.56 | 97.65 | 100.00 | 100.00 | 100.00 | 100.00 | 100.00 |
| Selenium sulfide | 96.17 | 98.54 | 100.00 | 100.00 | 100.00 | 100.00 | 100.00 |
| Zinc pyrithione | 86.6 | 95.71 | 100.00 | 100.00 | 100.00 | 100.00 | 100.00 |
| Chitinase | 10.67 | 16.15 | 23.85 | 35.38 | 44.62 | 61.99 | 63.8 |
| Chitosanase | 3.24 | 5.38 | 26.15 | 26.92 | 46.92 | 63.4 | 67.82 |
| Chitinase + Chitosanase (1:1) | 83.13 | 91.03 | 98.38 | 100.00 | 100.00 | 100.00 | 100.00 |
| β-glucanase | 2.6 | 4.15 | 10.75 | 13.24 | 21.1 | 30.76 | 52.21 |
| β-glucosidase | 10.56 | 18.16 | 32.25 | 38.6 | 43.44 | 59.71 | 72.4 |
| β-mannanase | 1.21 | 2.92 | 3.45 | 3.81 | 4.26 | 7.35 | 10.11 |
| Cellulase | 12.98 | 16.12 | 24.3 | 26.02 | 35.72 | 40.46 | 52.52 |
| Chitinase + β-glucanase (1:1) | 69.34 | 75.86 | 82.3 | 85.65 | 90.7 | 100.00 | 100.00 |
| β-mannanase + β-glucosidase + chitinase (1:1:1) | 95.62 | 98.07 | 100.00 | 100.00 | 100.00 | 100.00 | 100.00 |
| Chitosanase + β-glucosidase + chitinase (1:1:1) | 99.56 | 99.7 | 100.00 | 100.00 | 100.00 | 100.00 | 100.00 |
| Chitinase + cellulase (1:1) | 68.22 | 70.37 | 86.29 | 87.31 | 94.62 | 96.99 | 100.00 |
| Chitinase + β-glucosidase (1:1) | 82.61 | 84.99 | 87.83 | 89.86 | 92.16 | 93.70 | 100.00 |
| Chitinase + β-mannanase (1:1) | 76.02 | 79.57 | 80.11 | 80.67 | 82.90 | 83.21 | 84.62 |
| Chitosanase + β-glucosidase (1:1) | 79.23 | 85.90 | 87.76 | 93.66 | 99.08 | 99.98 | 100.00 |
| Chitosanase + β-glucanase (1:1) | 51.84 | 62.90 | 70.45 | 76.11 | 82.44 | 85.77 | 89.23 |

### LITERATURE

1. Byrd, A.L.; Belkaid, Y.; Segre, J.A. The human skin microbiome \\ Nat. Rev. Microbiol. 2018, 16, 143-155.
2. Oh, J., Byrd, A. L., Deming, C., Conlan, S., Kong, H. H., Segre, J. A., et al. (2014). Biogeography and individuality shape function in the human skin metagenome \\ Nature 514, 59-64.
3. Brandwein, M., Steinberg, D., and Meshner, S. (2016). Microbial biofilms and the human skin microbiome \\ NPJ Biofilms Microbiomes 2:3.
4. Rinaldi, F.; Pinto, D.; Marzani, B.; Rucco, M.; Sorbellini, E.; Giuliani, G. Human microbiome: What's new in scalp diseases \\ J. Transl. Sci. 2018, 4, 1-4.
5. Pinto, D.; Sorbellini, E.; Marzani, B.; Rucco, M.; Giuliani, G.; Rinaldi, F. Scalp bacterial shift in Alopecia areata \\ PLoS ONE 2019, 14.
6. Pinto, D.; Calabrese, F.M.; De Angelis, M.; Celano, G.; Giuliani, G.; Gobbetti, M.; Rinaldi, F. Predictive Metagenomic Profiling, Urine Metabolomics, and Human Marker Gene Expression as an Integrated Approach to Study Alopecia Areata \\ Front. Cell. Infect. Microbiol. 2020, 10, 146.
7. Nakatsuji, T.; Chiang, H.I.; Jiang, S.; Nagarajan, H.; Zengler, K.; Gallo, R.L. The microbiome extends to subepidermal compartments of normal skin \\ Nat. Commun. 2013, 4, 1431.
8. Saxena, R.; Mittal, P.; Clavaud, C.; Dhakan, D.B.; Hegde, P.; Veeranagaiah, M.M.; Saha, S.; Souverain, L.; Roy, N.; Breton, L.; et al. Comparison of Healthy and Dandruff Scalp Microbiome Reveals the Role of Commensals in Scalp Health \\ Front. Cell. Infect. Microbiol. 2018, 8, 346.
9. Grimshaw, S.G.; Smith, A.M.; Arnold, D.S.; Xu, E.; Hoptroff, M.; Murphy, B. The diversity and abundance of fungi and bacteria on the healthy and dandruff affected human scalp \\ PLoS ONE 2019, 14.
10. Clavaud, C.; Jourdain, R.; Bar-Hen, A.; Tichit, M.; Bouchier, C.; Pouradier, F.; El Rawadi, C.; Guillot, J.; Ménard-Szczebara, F.; Breton, L.; et al. Dandruff is associated with disequilibrium in the proportion of the major bacterial and fungal populations colonizing the scalp \\ PLoS ONE 2013, 8.
11. Wang, L.; Clavaud, C.; Bar-Hen, A.; Cui, M.; Gao, J.; Liu, Y.; Liu, C.; Shibagaki, N.; Guéniche, A.; Jourdain, R.; et al. Characterization of the major bacterial-fungal populations colonizing dandruff scalps in Shanghai, China, shows microbial disequilibrium \\ Exp. Dermatol. 2015, 24, 398-400.
12. Soares, R.C.; Camargo-Penna, P.H.; de Moraes, V.C.S.; De Vecchi, R.; Clavaud, C.; Breton, L.; Braz, A.S.K.; Paulino, L.C. Dysbiotic bacterial and fungal communities not restricted to clinically affected skin sites in dandruff \\ Front. Cell. Infect. Microbiol. 2020, 6, 157.
13. Xu, Z.; Wang, Z.; Yuan, C.; Liu, X.; Yang, F.; Wang, T.; Wang, J.; Manabe, K.; Qin, O.; Wang, X.; et al. Dandruff is associated with the conjoined interactions between host and microorganisms \\ Sci. Rep. 2016, 6, 24877.
14. Rinaldi, F.; Trink, A.; Papale, A.; Giuliani, G.; Pinto, D. Clinical Translation of Microbiome Research in Alopecia Areata: A New Perspective? \\ Cosmetics. 2022, 9, 55.
15. Lin, Q., Panchamukhi, A., Li, P. et al. Malassezia and Staphylococcus dominate scalp microbiome for seborrheic dermatitis \\ Bioprocess Biosyst Eng 44, 965-975 (2021).
16. Schommer N.N., Gallo R.L. Structure and function of the human skin microbiome \\ Trends Microbiol. 2013; 21(12), 660-8.
17. Narshana M and Ravikumar P: An overview of dandruff and novel formulations as a treatment strategy \\ Int J Pharm Sci & Res. 2018; 9(2): 417-31.
18. Gilbertson K, Jarrett R, Bayliss SJ and Berk DR: Scalp discoloration from selenium sulfide shampoo: a case series and review of the literature \\ Pediatric dermatology 2012; 29(1): 84-8.
19. Couto FM, Nascimento SC, Júnior SF, Silva VK, Leal AF and Neves RP: Antifungal activity of the piroctoneolamine in experimental intra-abdominal candidiasis \\ Springer Plus 2016; 5(1): 468.
20. Mangion, S.E.; Holmes, A.M.; Roberts, M.S. Targeted Delivery of Zinc Pyrithione to Skin Epithelia \\ Int. J. Mol. Sci. 2021, 22, 9730.
21. Israa A. Al-Temimay 1, Alaa M. Hasan. Isolation and identification of fungi from the droppings of some poultry and some detergents effect on some of them \\ Iraqi Journal of Science, 2016, Vol. 57, No.4B, pp: 4362 -2640.
22. Shibata, N., Saitoh, T., Tadokoro, Y., & Okawa, Y. (2009). The cell wall galactomannan antigen from Malassezia furfur and Malassezia pachydermatis contains -1,6-linked linear galactofuranosyl residues and its detection has diagnostic potential \\ Microbiology, 155(10), 3420-3429.
23. Ravindra Pal Singh, Sivasubramanian Rajarammohan, Raksha Thakur & Mohsin Hassan. Linear and branched β-Glucans degrading enzymes from versatile Bacteroides uniformis JCM 13288T and their roles in cooperation with gut bacteria \\ Gut Microbes, 12: 1, (2020).
24. Sakamoto, Y., Nakade, K., Sato, S., Yoshida, K., Miyazaki, K., Natsume, S., & Konno, N. (2017). Lentinula edodes Genome Survey and Postharvest Transcriptome Analysis \\ Applied and Environmental Microbiology, 83(10).
25. Hua Zhang, Steven H. Neau. In vitro degradation of chitosan by a commercial enzyme preparation: effect of molecular weight and degree of deacetylation \\ Biomaterials, V. 22, 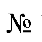 12, 2001, Pages 1653-1658.
26. Hamid R, Khan MA, Ahmad M, Ahmad MM, Abdin MZ, Musarrat J, Javed S. Chitinases: An update \\ J Pharm Bioallied Sci. 2013 Jan;5(1):21-9.
27. Nidheesh Thadathil, Suresh Puthanveetil Velappan. Recent developments in chitosanase research and its biotechnological applications: A review \\ Food Chemistry. Volume 150, 2014, Pages 392-399.
28. Guého E, Midgley G, Guillot J. The genus Malassezia with description of four new species. Antonie Van Leeuwenhoek. 1996, 69(4):337-55.
29. Anita Singh, Somvir Bajar, Arti Devi, Deepak Pant. An overview on the recent developments in fungal cellulase production and their industrial applications \\ Bioresource Technology Reports, Volume 14, 2021, 100652.
30. Yu Cao, Huimin Tan. Effects of cellulase on the modification of cellulose \\ Carbohydrate Research, Volume 337, Issue 14, 2002, Pages 1291-1296.
31. Barry V. McCleary. β-d-Mannanase \\ Methods in Enzymology, Academic Press, Volume 160, 1988, Pages 596-610.
32. Abosereh, NivienA & Ismail, SihamA & Khattab, OmK.H & Nour, ShaimaaA & Abo-Elnasr, AmanyA & Hashem, AmalM. (2019). Genetic improvement of fungal β-mannanase and its molecular differentiation \\ Egyptian Pharmaceutical Journal. 18. 403.
33. Antoni Planas. Bacterial 1,3-1,4-β-glucanases: structure, function and protein engineering \\ Biochimica et Biophysica Acta (BBA) - Protein Structure and Molecular Enzymology, Volume 1543, Issue 2, 2000, Pages 361-382.
34. V G Dzhavakhiya, O.L. Ozeretskovskaya, S.V. Zinovyeva. Chapter 10 - Immune response. In Studies in Plant Science \\ Comprehensive and Molecular Phytopathology, Elsevier, 2007, Pages 265-314.
35. Srivastava, N.; Rathour, R.; Jha, S.; Pandey, K.; Srivastava, M.; Thakur, V.K.; Sengar, R.S.; Gupta, V.K.; Mazumder, P.B.; Khan, A.F.; et al. Microbial Beta Glucosidase Enzymes: Recent Advances in Biomass Conversation for Biofuels Application. Biomolecules 2019, 9, 220.
36. Thomas Stalhberger Catherine Simenel§, Ce' cile Clavaud, Vincent G. H. Eijsink, Roland Jourdain, Muriel Delepierre, Jean-Paul Latge, Lionel Breton and Thierry Fontaine. Chemical organization of the cell wall polysaccharide core of Malassezia restricta \\ J Biol Chem. 2014 May 2;289(18): 12647-56. doi: 10.1074/jbc.M113.547034. Epub 2014 Mar 13.
37. Ergin C., Kurt O., Turkoglu M., Sevinc H., Akbaba G. Evaluation of novel cosmetic shampoo formulations against Malassezia species: Preliminary results of anti-dandruff shampoo formulations \\ Journal of Cosmetic Dermatology 2024 23:6 (2078-2083).
38. CN115607656A·(BAIKUIRUI TIANJIN BIOTECHNOLOGY CO LTD) 2023-01-17.

## Claims

1. The use of the following combination of enzymes
a) chitinase
in combination with at least one of the following enzymes:
i) chitosanase;
ii) β-glucanase;
iii) β-glucosidase;
iv) β-mannanase;
v) cellulase;
wherein said combination of chitinase and said further enzyme selected from i)-v) are taken at a mass ratio 1.0 : 0.5-1.5, preferably 1.0 : 0.7-1.3, further preferably 1.0 : 0.9-1.1, even more preferably 1.0 : 1.0; or
b) chitosanase
in combination with at least one of the following enzymes:
i) chitinase;
ii) β-glucanase;
iii) β-glucosidase;
iv) β-mannanase;
v) cellulase;
wherein said combination of chitosanase and said further enzyme selected from i)-v) are taken at a mass ratio 1.0 : 0.5-1.5, preferably 1.0 : 0.7-1.3, further preferably 1.0 : 0.9-1.1, even more preferably 1.0 : 1.0;
in the manufacture of a hair care and/or scalp care composition.

2. Non-medical use of a hair care and/or scalp care composition, said composition comprising the following combination of enzymes
a) chitinase
in combination with at least one of the following enzymes:
i) chitosanase;
ii) β-glucanase;
iii) β-glucosidase;
iv) β-mannanase;
v) cellulase;
wherein said combination of chitinase and said further enzyme selected from i)-v) are taken at a mass ratio 1.0 : 0.5-1.5, preferably 1.0 : 0.7-1.3, further preferably 1.0 : 0.9-1.1, even more preferably 1.0 : 1.0; or
b) chitosanase
in combination with at least one of the following enzymes:
i) chitinase;
ii) β-glucanase;
iii) β-glucosidase;
iv) β-mannanase;
v) cellulase;
wherein said combination of chitosanase and said further enzyme selected from i)-v) are taken at a mass ratio 1.0 : 0.5-1.5, preferably 1.0 : 0.7-1.3, further preferably 1.0 : 0.9-1.1, even more preferably 1.0 : 1.0;
for application on a subject's hair and/or scalp.

3. The non-medical use of claim 2, wherein said composition is for the treatment of the hair and/or scalp of a subject suffering from dandruff.

4. The non-medical use of claim 3, wherein said dandruff is caused by fungi of the *Malassezia* species.

5. The non-medical use of claim 4, wherein said *Malassezia* species comprise *M. furfur, M. sympodialis, M. obtusa, M. globosa, M. restricta, M. slooffiae* and *M. pachydermatis,* preferably *M. furfur, M. globosa, M. restricta,* further preferably *M. furfur CBS 1878, M. restricta CBS 7877, M. globosa CBS 7966.*

6. A hair care and/or scalp care composition, said composition comprising the following combination of enzymes
a) chitinase
in combination with at least one of the following enzymes:
i) chitosanase, preferably further comprising β-glucosidase;
ii) β-glucanase;
iii) β-glucosidase, preferably further comprising β-mannanase;
iv) β-mannanase;
v) cellulase;
wherein said combination of chitinase and said further enzyme selected from i)-v) are taken at a mass ratio 1.0 : 0.5-1.5, preferably 1.0 : 0.7-1.3, further preferably 1.0 : 0.9-1.1, even more preferably 1.0 : 1.0; or
b) chitosanase in combination with at least one of the following enzymes:
i) chitinase;
ii) β-glucanase;
iii) β-glucosidase;
iv) β-mannanase;
v) cellulase;
wherein said combination of chitosanase and said further enzyme selected from i)-v) are taken at a mass ratio 1.0 : 0.5-1.5, preferably 1.0 : 0.7-1.3, further preferably 1.0 : 0.9-1.1, even more preferably 1.0 : 1.0;
for use in the treatment of dandruff and/ or a disease selected from *Pityriasis capitis* and *Pityriasis simplex capillitia.*

7. The composition for use of claim 6, wherein said dandruff and/or disease is caused by fungi of the *Malassezia* species.

8. The composition for use of claim 7, wherein said *Malassezia* species comprise *M. furfur, M. sympodialis, M. obtusa, M. globosa, M. restricta, M. slooffiae* and *M. pachydermatis,* preferably *M. furfur, M. globosa, M. restricta,* further preferably *M. furfur CBS 1878, M. restricta CBS 7877, M. globosa CBS 7966.*

9. The use or composition for use of any of the preceding claims, wherein said composition comprises any of the combination of said enzymes, including one of the following enzyme combinations:
i) chitinase and chitosanase;
ii) chitinase and β-glucanase;
iii) chitinase and β-glucosidase;
iv) chitinase and β-mannanase;
v) chitinase and cellulase;
vi) chitosanase and β-glucanase;
vii) chitosanase and β-glucosidase;
viii) chitosanase and β-mannanase;
ix) chitosanase and cellulase;
x) chitinase, β-glucanase and β-glucosidase;
xi) chitinase, β-glucanase and β-mannanase;
xii) chitinase, β-glucanase and cellulase;
xiii) chitosanase, β-glucanase and β-glucosidase;
xiv) chitosanase, β-glucanase and β-mannanase;
xv) chitosanase, β-glucanase and cellulase;
xvi) chitinase, chitosanase and β-glucanase;
xvii) chitinase, chitosanase and β-glucosidase;
xviii) chitinase, chitosanase and β-mannanase;
xix) chitinase, chitosanase and cellulase;
xx) chitinase, chitosanase, β-glucanase and β-glucosidase;
xxi) chitinase, chitosanase, β-glucanase and β-mannanase;
xxii) chitinase, chitosanase, β-glucanase and cellulase;
xxiii) chitinase, chitosanase, β-glucanase, β-glucosidase and β-mannanase;
xxiv) chitinase, chitosanase, β-glucanase, β-glucosidase and cellulase;
xxv) chitinase, chitosanase, β-glucanase, β-glucosidase, β-mannanase and cellulase.

10. The use or composition for use of any of the preceding claims,
said chitinase having the CAS 9001-06-3 registration number;
said chitosanase having the CAS 51570-20-8 registration number;
said β-glucanase having the CAS 62213-14-3 registration number;
said β-glucosidase having the CAS 9001-22-3 registration number;
said β-mannanase having the CAS 37288-54-3 registration number;
said cellulase having the CAS 9012-54-8 registration number.

11. The use or composition for use of any of the preceding claims, wherein said composition is formulated for application on a subject's hair and/or scalp.

12. The use or composition for use of claim 11, wherein said composition is a formulation selected from the following: liquid, aerosol, spray, foam, suspension, solution, tincture, cream, paste, lotion, ointment, gel, balsam.

13. The use or composition for use of claim 11 and/or 12, wherein, wherein said formulations are scalp skin and/or hair adhesive formulations, including shower gel and shampoo for scalp and/or hair care.
